(19) Europäisches Patentamt
European Patent Office
Office européen des brevets

(11) Veröffentlichungsnummer: **0 324 984**
**B1**

(12) # EUROPÄISCHE PATENTSCHRIFT

(45) Veröffentlichungstag der Patentschrift: **31.10.90**

(51) Int. Cl.[5]: **B01J 23/46**, B01J 23/58, C07C 209/68

(21) Anmeldenummer: **88121825.9**

(22) Anmeldetag: **29.12.88**

(54) **Verfahren zur Herstellung eines Gemisches aus Cyclohexylamin und Dicyclohexylamin unter Einsatz eines Ruthenium-Katalysators.**

(30) Priorität: **22.01.88 DE 3801756**

(43) Veröffentlichungstag der Anmeldung: **26.07.89 Patentblatt 89/30**

(45) Bekanntmachung des Hinweises auf die Patenterteilung: **31.10.90 Patentblatt 90/44**

(84) Benannte Vertragsstaaten: **BE CH DE FR GB IT LI**

(56) Entgegenhaltungen:
**DE-A-1 106 319**
**GB-A-2 023 020**
**US-A-3 697 449**
**US-A-4 070 399**
**US-A-4 496 666**

**CHEMICAL ABSTRACTS, Band 85, Nr. 23, 6. Dezember 1976, Zusammenfassung Nr. 176953g, Columbus, Ohio, US;**

(73) Patentinhaber: **BAYER AG, D-5090 Leverkusen 1 Bayerwerk(DE)**

(72) Erfinder: **Immel, Otto, Dr., Immenhofweg 26, D-4150 Krefeld(DE)**
Erfinder: **Schwarz, Hans-Helmut, Dr., Ratherstrasse 90, D-4150 Krefeld 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 324 984 B1

**Beschreibung**

Die Erfindung betrifft ein Verfahren zur Herstellung eines Gemisches aus gegebenenfalls substituiertem Cyclohexylamin und gegebenenfalls substituiertem Dicyclohexylamin durch katalytische Hydrierung von gegebenenfalls substituiertem Anilin unter Einsatz eines Ruthenium-Katalysators.

Es ist bekannt, Cyclohexylamin und andere kernhydrierte Aminoverbindungen durch katalytische Hydrierung von Anilin und anderen aromatischen Aminoverbindungen herzustellen. Als Katalysatoren sind hierfür bekannt: Kobalt-Katalysatoren, die einen basischen Zusatz enthalten (GB 969 542), Raney-Kobalt (JP 68/03180), Ruthenium-Katalysatoren (DE-AS 11 06 319), mit Alkalimetallverbindungen dotierte Ruthenium-Katalysatoren (US 3.636.108) oder Nickelkatalysatoren (DE-PS 805 518).

Die meisten der genannten Verfahren werden unter Druck betrieben und ergeben hauptsächlich Cyclohexylamin neben nur wenig Dicyclohexylamin. Das Dicyclohexylamin wird daher vielfach durch andere Verfahren hergestellt, so beispielsweise durch Druckhydrierung von Diphenylamin unter Verwendung eines Ruthenium-Katalysators (DE-AS 11 06 319). Weiterhin entsteht Dicyclohexylamin bei der Umsetzung von Cyclohexanon mit Cyclohexylamin in Gegenwart eines Palladium/Kohle-Katalysators unter einem Wasserstoffdruck von etwa 4 bar (FR 1.333.692). Das Verfahren der genannten DE-PS 805 518 ist hauptsächlich auf die Gewinnung von Dicyclohexylamin ausgerichtet, arbeitet jedoch mit umständlichen Nebenproduktrückführungen.

Weitere Nachteile der genannten Verfahren bestehen in zum Teil beträchtlichen Mengen an Cyclohexan-Abfallprodukt sowie in der unbefriedigenden Standzeit der eingesetzten Katalysatoren. Es bestand daher der Wunsch, ein in technischem Maßstab brauchbares Verfahren zu entwickeln, bei welchem der Verlust durch die Bildung von Cyclohexan zurückgedrängt wird und die Standzeit des verwendeten Katalysators verbessert ist, sowie ein Verfahren zu entwickeln, bei dem gemeinsam Cyclohexylamin und Dicyclohexylamin in Mengen gebildet werden, die je nach dem Bedarf der beiden genannten Stoffe variabel sind.

Überraschenderweise wurde gefunden, daß die genannten Anforderungen durch den Einsatz des im folgenden gekennzeichneten Ruthenium-Trägerkatalysator erfüllt werden.

Die Erfindung betrifft ein Verfahren zur Herstellung eines Gemisches aus gegebenenfalls substituiertem Cyclohexylamin und gegebenenfalls substituiertem Dicyclohexylamin durch Hydrierung von gegebenenfalls substituiertem Anilin in Gegenwart eines Ruthenium-Katalysators, das dadurch gekennzeichnet ist, daß man einen Ruthenium und Palladium enthaltenden Katalysator auf einem Träger, der die Edelmetalle in einer Gesamtmenge von 0,05–5 Gew.-%, bevorzugt 0,1–4 Gew.-%, besonders bevorzugt 0,1–3 Gew.-% und in einem Gewichtsverhältnis Ruthenium zu Palladium wie 1:9–9:1, bevorzugt 2:8–8:2, besonders bevorzugt 3:7–7:3, enthält, und der weiterhin 0,1–10 Gew.-%, bevorzugt 0,2–5 Gew.-%, einer alkalisch reagierenden Alkalimetallverbindung enthält, wobei alle Prozentsätze auf das Gesamtgewicht des Katalysators bezogen sind, einsetzt und die Hydrierung bei 150–220°C und einem Druck von 0,5–10 bar, bevorzugt 0,5–4 bar, besonders bevorzugt 0,7–2 bar durchführt.

Die erfindungsgemäß einzusetzenden Katalysatoren sind demnach hauptsächlich durch die Kombination von Ruthenium mit Palladium ausgezeichnet. Solche Katalysatoren haben gegenüber den nur Ruthenium enthaltenden Katalysatoren eine beträchtlich erhöhte Standzeit, was für ihren Einsatz im technischen Verfahren unerläßlich ist.

Als basische Alkalimetallverbindungen für die erfindungsgemäß einzusetzenden Katalysatoren sind beispielsweise als Oxids, Hydroxide, Alkoholats oder Salze schwacher Säuren von Lithium, Natrium, Kalium, Rubidium oder Cäsium, bevorzugt als Hydroxide, Alkoholats oder Salze schwacher Säuren von Lithium, Natrium oder Kalium, besonders bevorzugt von Natrium oder Kalium. Schwache Säuren sind beispielsweise Kohlensäure, Essigsäure, Ameisensäure und anders Carbonsäuren, deren Alkalisalze alkalisch reagieren und sind in jedem Falls solche, als frei sind von Stickstoff, Halogen, Schwefel und anderen als Gifte für Hydrierkatalysatoren geltenden Elementen. Alkoholate sind beispielsweise solche des Methanols, Ethanols, Propanols, Butanols und anderer Alkohole.

Die genannten aktiven Materialien der Katalysatoren sind auf einem Träger angeordnet. Beispiele für solche Träger sind Aluminiumoxid, Aluminiumspinell, Aktivkohle, Kieselgur, Bentonit, Bimsstein, Silicagel, $ZrO_2$, $TiO_2$, ZnO, MgO sowie Oxide der Seltenen Erden.

Die Katalysatoren sind bevorzugt auf einem Träger aus $Al_2O_3$ oder einem Aluminiumspinell angeordnet. Als $Al_2O_3$ kommen insbesondere die α- und die γ-Modifikationen in Frage. Aluminiumspinelle sind Verbindungen der Formel

$Me(II)Al_2O_4$ bzw. $Me(I)AlO_2$

in denen

Me(II) ein zweiwertiges Metallkation des Eisens, Zinks, Nickels, Kupfers, Kobalts, Cadmiums, Magnesiums oder anderer, bevorzugt des Magnesiums, und Me(I) ein einwertiges Kation, beispielsweise Lithium (Lithium-Aluminium-Spinell), ist. Das Aluminium in den Spinellen kann teilweise durch dreiwertiges Eisen, Chrom oder Mangan ersetzt sein.

Zur Herstellung der Katalysatoren kann so vorgegangen werden, daß auf einen der genannten Träger, bevorzugt auf ein $Al_2O_3$ oder einen Aluminiumspinell in Form von Strangpreßlingen, Pillen oder Kugeln mit Abmessungen von etwa 2–10 nun die genannten Edelmetalle in Form geeigneter Salze, sowie die alkalisch reagierenden Alkalimetallverbindungen in getrennten Vorgängen aufgetragen werden und

nach jedem getrennten Auftragen getrocknet wird. Das Trocknen geschieht in bekannter Weise, beispielsweise bei 100–140°C und vermindertem bis normalem Druck, etwa bei 1–1000 mbar, vielfach 10–500 mbar, beispielsweise bei Wasserstrahlvakuum. In bevorzugter Weise wird hierbei von wäßrigen Lösungen zum Auftragen ausgegangen. Grundsätzlich kommen jedoch auch alkoholische Lösungen oder Lösungen in niederen Carbonsäuren oder niederen Aminen in Frage, sofern die vorgesehenen Salze der Edelmetalle bzw. die basischen Alkalimetallverbindungen darin löslich sind.

Geeignete Salze der Edelmetalle sind beispielsweise ihre Chloride, Nitrate, Acetate. Unabhängig davon, ob das Auftragen der alkalisch reagierenden Alkalimetallverbindungen vor oder nach dem Auftragen der Edelmetallsalze durchgeführt wird, werden die Edelmetalle beim Zusammentreffen mit den alkalisch reagierenden Alkalimetallverbindungen auf dem Träger in Form ihrer Oxide oder Hydroxide ausgefällt. Nach der abschließenden Trocknung steht der Katalysator zur Verfügung. Er wird in bevorzugter Weise im Reaktor vor seinem Einsatz durch Behandlung mit Wasserstoff bei erhöhter Temperatur aktiviert. Eine solche erhöhte Temperatur liegt beispielsweise bei 120–400°, bevorzugt bei 150–380°C.

Die Katalysatoren können in hervorragender Weise zur Kernhydrierung von Anilin verwendet werden. Diese Hydrierung kann in überraschender Weise in einer Gasphasenreaktion bei vermindertem Druck, Normaldruck oder nur mäßig erhöhtem Druck, also im wesentlichen drucklos, durchgeführt werden. Damit sind als mechanischen Anforderungen an die erforderlichen technischen Apparaturen und damit ihr Herstellungspreis erheblich zu erniedrigen. Das bei dieser Kernhydrierung von Anilin entstehende Gemisch von Cyclohexylamin und Dicyclohexylamin kann weiterhin in überraschender Weise in seinem Verhältnis zueinander durch die Wahl der Reaktionstemperatur gesteuert werden.

Im Rahmen des erfindungsgemäßen Verfahrens wird der Anteil an Dicyclohexylamin erhöht, wenn eine niedrigere Temperatur innerhalb des genannten Bereiches gewählt wird.

In einer dem Fachmann bekannten Weise wird weiterhin der untere Teil des gesamten Temperaturbereiches vornehmlich niedrigeren Drücken im genannten Bereich zugeordnet und umgekehrt.

Im erfindungsgemäßen Verfahren wird eine Katalysatorbelastung von 0,05–2 kg, bevorzugt 0,1–0,5 kg Anilin pro Liter Katalysator und Stunde eingestellt.

Eine geringe Veränderung des erzielten Anteils an Dicyclohexylamin durch etwa veränderte Aktivität des Katalysators im Laufe längerer Reaktionsperioden kann durch ein geringes Nachstellen der Reaktionstemperatur oder der anderen genannten Parameter ausgeglichen werden. Das gewünschte Verhältnis von Cyclohexylamin und Dicyclohexylamin kann anhand der Analytik des Reaktionsgemisches einfach verfolgt werden.

Als Einsatzmaterialien kommen im Sinne der folgenden Reaktionsgleichung Anilin und substituierte Aniline in Betracht, die zu den korrespondierenden Cyclohexylaminen und Dicyclohexylaminen umgesetzt werden:

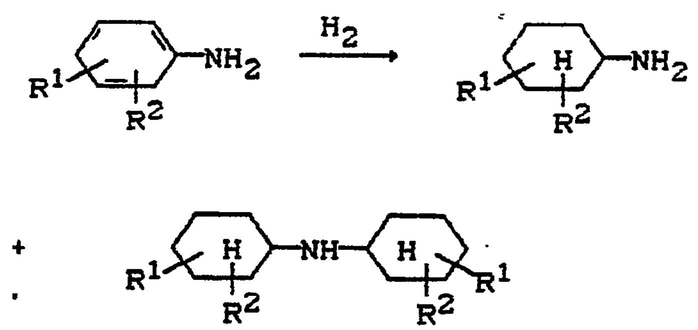

+

Die Reste $R^1$ und $R^2$ haben unabhängig voneinander die Bedeutung von Wasserstoff, $C_1$-$C_4$-Alkyl oder $C_1$-$C_4$-Alkoxy. Beispiele für die genannten Alkyl- bzw. Alkoxysubstituenten sind: Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl bzw. Methoxy. In weiterhin bevorzugter Weise hat einer der Substituenten $R^1$ und $R^2$ die Bedeutung Wasserstoff, während der andere Substituent Alkyl bzw. Alkoxy im genannten Umfang bedeutet. In besonders bevorzugter Weise richtet sich das erfindungsgemäße Verfahren auf die Kernhydrierung von nicht-substituiertem Anilin.

Im erfindungsgemäßen Verfahren werden 1-100 Normliter Wasserstoff pro 1 g Anilin, bevorzugt 1-50 Normliter $H_2$, eingesetzt.

Cyclohexylamine und Dicyclohexylamine des genannten Bedeutungsumfanges finden Verwendung zur Herstellung von Alterungsschutzmitteln für Kautschuke und Kunststoffe, als Korrosionsschutzmittel, sowie als Vorprodukte für Pflanzenschutzmittel und Textilhilfsmittel.

Das im Bereich niedriger Drücke durchführbare erfindungsgemäße Verfahren zur Herstellung eines Gemisches aus gegebenenfalls substituiertem Cyclohexylamin und gegebenenfalls substituiertem Dicyclohexylamin eignet sich weiterhin in hervorragender Weise zur direkten Kombination mit einem ebenfalls im Bereich niedriger Drücke durchführbaren Verfahren zur Herstellung von gegebenenfalls substituiertem Diphenylamin aus den erfindungsgemäß herstellbaren gegebenenfalls substituierten Dicyclohexylaminen.

Ein solches Verfahren zur Herstellung von Diphenylamin kann bei 250-450°C und einem Druck von 1-10 bar durchgeführt werden, wobei man als Katalysator einen solchen einsetzt, der Rhodium und mindestens ein anderes Platinmetall aus der Gruppe Palladium, Platin und Iridium enthält, welche auf einem mit Chrom und Mangan behandelten Träger aus der Gruppe von $Al_2O_3$ und Aluminiumspinell angeordnet sind. Ein solcher Katalysator enthält die Edelmetalle in einer Gesamtmenge von 0,05-5 Gew.-%, bevorzugt 0,05-4 Gew.-% besonders bevorzugt 0,1-3 Gew.-%; der Gewichtsanteil des Rhodiums an der Gesamtmenge der Edelmetalle beträgt 10-90 %, bevorzugt 15-80 %, besonders bevorzugt 20-70 %. Ein solcher Katalysator enthält weiterhin Zusätze von 1-6 Gew.-% eines Alkalihydroxids und 1-6 Gew.-% eines Alkalimetallsulfats. Alle genannten Prozentsätze sind auf das Gesamtgewicht des Katalysators bezogen.

Bei der Kombination des erfindungsgemäßen Verfahrens und des zuletztgenannten Verfahrens zur Herstellung von Diphenylamin wird das erfindungsgemäß erhaltene Gemisch aus gegebenenfalls substituiertem Cyclohexylamin und gegebenenfalls substituiertem Dicyclohexylamin eingesetzt; dieses einzusetzende Gemisch kann weiterhin N-Cyclohexyl-anilin enthalten. Dieses dem erfindungsgemäßen Verfahren entstammende Gemisch für die Herstellung von Diphenylamin wird mit Hilfe eines inerten Trägergasstromes, beispielsweise eines $N_2$-oder $H_2$-Stromes, an den für die Diphenylamin-Herstellung genannten, Rhodium enthaltenden Katalysator gebracht. Damit ist es möglich, den aus der erfindungsgemäßen Herstellung von Cyclohexylamin und Dicyclohexylamin stammenden überschüssigen Wasserstoff als ein solches Trägergas zu benutzen.

In weiterhin bevorzugter Weise können die Stufen der erfindungsgemäßen Hydrierung von Anilin zum Gemisch Cyclohexylamin/Dicyclohexylamin und die Stufe der Diphenylamin-Herstellung apparativ so zusammengefaßt werden, daß in einem Reaktor mit zwei hintereinanderliegenden Schichten der oben beschriebenen Ruthenium- bzw. Rhodium-Katalysatoren Anilin in Gegenwart von Wasserstoff zunächst erfindungsgemäß umgesetzt wird und dann das dabei entstehende Gemisch direkt an der zweiten Katalysatorschicht zu Diphenylamin weiter umgesetzt wird. Das nach der Abtrennung des gewünschten Diphenylamins verbleibende Reaktionsgemisch kann dem erfindungsgemäßen Verfahren wieder zugeführt werden. Es kann bei einer solchen Rückführung sinnvoll sein, einen Teil des bei der Hydrierung von Anilin zum Gemisch Cyclohexylamin/Dicyclohexylamin entstehenden Ammoniaks auszuwaschen oder durch Kompression auszukondensieren. Eine solche Entfernung von Ammoniak ist dem Fachmann im Prinzip bekannt.

<u>Beispiel 1</u>

500 g eines handelsüblichen $\gamma$-$Al_2O_3$ mit einer spezifischen Oberfläche von 350 $m^2/g$ und einen Kugeldurchmesser von 2 bis 6 mm wurden mit einer Lösung getränkt, die aus 20 g NaOH und 170 g Wasser hergestellt wurde. Das getränkte $Al_2O_3$ wurde bei 120°C im Wasserstrahlvakuum getrocknet. 100 g des so behandelten $Al_2O_3$ wurden mit einer Lösung getränkt, die aus 1,50 g $RuCl_3$, 1,17 g $PdCl_2$ und 30 g Wasser hergestellt worden war. 30 ml (23,1 g) des bei 120°C im Wasserstrahlvakuum nochmals getrockneten Katalysators werden 24 Stunden in einem Wasserstoffstrom von 10 l/h bei 200°C aktiviert. Durch diesen Katalysator, der sich in einen senkrecht angeordneten 17 mm weiten Reaktionrohr befand, wurden pro Stunde 4,07 g Anilin zusammen mit 20 l Wasserstoff geleitet. Im Laufe einer Versuchsdauer von über 4000 Stunden wurde die Reaktionstemperatur zwischen 160 und 200°C variiert. Das entstandene Reaktionsprodukt wurde kondensiert und in verschiedenen Zeitabständen analysiert. Dabei wurden in Abhängigkeit von den Betriebsstunden des Katalysators und der Reaktionstemperatur folgende Zusammensetzung des Reaktionsproduktes gefunden:

| Versuchsdauer: | 612 h | 3043 h | 3272 h | 4175 h |
|---|---|---|---|---|
| Cyclohexyl-amin | 19,3 | 21,6 | 20,5 | 17,2 % |
| Dicyclohexyl-amin | 80,3 | 66,8 | 76,2 | 81,9 % |
| N-Cyclohexyl-anilin | 0,1 | 2,0 | 0,8 | - % |
| Anilin | 0,1 | 9,3 | 2,4 | 0,8 % |
| Nebenprodukte | 0,2 | 0,3 | 0,1 | 0,1 % |
| Reaktionstemp. | 160 | 200 | 180 | 160° C |

Beispiel 2

In diesem Beispiel diente Anilin als Ausgangsverbindung für die Herstellung von Diphenylamin. Benutzt wurden hierbei zwei übereinanderstehende Reaktionsrohre (innerer Durchmesser = 17 mm), die jeweils mit verschiedenen Katalysatoren gefüllt waren und auch auf verschiedenen Temperaturen gehalten wurden. In dem ersten (oberen) Reaktionsrohr befanden sich 30 ml eines Katalysators aus Ru (0,5 %) und Pd (0,5 %) auf $Al_2O_3$, der mit 4 % NaOH versetzt worden war.
Diese Katalysatorschicht wurde auf 180°C gehalten.
Dieser Katalysator war wie folgt hergestellt worden:
500 g eines handelsüblichen $\gamma$-$Al_2O_3$ (Kugeldurchmesser: 2-5 mm) mit einer spezifischen Oberfläche von 350 $m^2/g$ wurde mit einer Lösung von 20 g NaOH in 170 ml Wasser getränkt und anschließend getrocknet. 100 g des so behandelten $Al_2O_3$ wurde mit einer Lösung von 2,5 g $RuCl_3$ und 0,83 g $PdCl_2$ in 30 ml Wasser getränkt, anschließend bei 120°C getrocknet und danach 2 Std. bei 250°C im Wasserstoffstrom aktiviert.

Das Reaktionsrohr mit dem in dieser Art hergestellten Katalysator war mit einem zweiten Rohr verbunden, in dem sich 30 ml eines Katalysators befanden, der wie folgt hergestellt worden war: 50 g eines mit Chrom and Mangan gemäß EP-Anmeldung 0 208 933, Beispiel 1, beaufschlagten $\gamma$-$Al_2O_3$ wurden in einem Rundkolben mit einer Lösung von 0,66 g $RhCl_3$ und 0,83 g $H_2PtCl_6$ in 15 ml Wasser gleichmäßig getränkt. Die feuchten Katalysatorpellets wurden bei 120°C getrocknet und danach mit einer Lösung von 1,46 g NaOH in 15 ml Wasser erneut getränkt und wieder getrocknet. Anschließend wurden die Pellets mit einer Lösung von 1,5 g $K_2SO_4$ in 15 ml Wasser nochmals getränkt und wieder getrocknet. Dieser Katalysator wurde auf einer Temperatur von 380°C gehalten. In die so hintereinandergeschalteten Reaktionsrohre wurden im Verlauf von 21,5 h 90 g Anilin zusammen mit 10 l $H_2$/h geleitet. Das aus dem zweiten Reaktionsrohr ausströmende Reaktionsprodukt wurde kondensiert und analysiert. Die Analyse ergab folgende Zusammensetzung:

| | |
|---|---|
| Diphenylamin: | 60,4 % |
| Cyclohexylamin: | 0,4 % |
| N-Cyclohexylanilin: | 6,3 % |
| Anilin: | 32,3 % |
| Nebenprodukte: | Rest |

Das nach Abtrennung des Diphenylamins verbleibende Gemisch wurde recyclisiert.
Das nicht kondensierte Abgas wurde nach Entfernung eines Teils des $NH_3$ ebenfalls recyclisiert.

Beispiel 3

200 g eines handeslüblichen $\gamma$-$Al_2O_3$ mit einer spezifischen Oberfläche von 350 $m^3/g$ und einem Kugeldurchmesser von 2-6 mm wurden mit einer Lösung, die aus 3,0 g $RuCl_3$ und 49 g Wasser hergestellt wurde, getränkt. Der so imprägnierte Katalysatorträger wurde bei 120°C unter Wasserstrahlvakuum getrocknet und anschließend 3 Stunden bei 350°C im Wasserstoffstrom reduziert. Danach wurde der Katalysator mit einer Lösung getränkt, die aus 8 g NaOH und 49 g Wasser hergestellt wurde.

40 ml (32,6 g) des bei 120°C im Wasserstrahlvakuum getrockneten Katalysators wurden zur Hydrierung von Anilin in der Gasphase eingesetzt. Es wurden pro Stunde 4,22 g Anilin zusammen mit 20 l $H_2$ durch die Katalysatorschicht bei 180°C geleitet. Das in 324 Stunden erzeugte Reaktionsprodukt hatte die unter Spalte A aufgegebene Zusammensetzung:

| | A | B |
|---|---|---|
| Cyclohexylamin | 16,7 | 35,0 |
| Dicyclohexylamin | 0,9 | 63,8 |
| N-Cyclohexylanilin | 5,9 | 0,6 |
| Anilin | 75,9 | 0,3 |
| Nebenprodukte | 0,6 | 0,3 |

Die unter Spalte B angeführte Produktzusammensetzung erbrachte hingegen 40 ml (32,6 g) eines Katalysators, der in völlig gleicher Weise hergestellt wurde, aber eine Kombination von Ru mit Pd enthielt. Zu seiner Herstellung wurden 200 g des gleichen $Al_2O_3$ mit einer wäßrigen Lösung getränkt, die 2,35 g $PdCl_2$ und 3,0 g $RuCl_2$ enthielt. Die weitere Herstellung des Katalysators war völlig identisch mit der des Ru-Katalysators, der zunächst benutzt wurde und das unter A angeführte Hydrierergebnis brachte.

## Patentansprüche

1. Verfahren zur Herstellung eines Gemisches aus gegebenenfalls substituiertem Cyclohexylamin und gegebenenfalls substituiertem Dicyclohexylamin durch Hydrierung von gegebenenfalls substituiertem Anilin in Gegenwart eines Rutheniumkatalysators, dadurch gekennzeichnet, daß man einen Ruthenium und Palladium enthaltenden Katalysator auf einem Träger, der die Edelmetalle in einer Gesamtmenge von 0,05–5 Gew.-% und einem Gewichtsverhältnis Ruthenium zu Palladium wie 1:9–9:1 enthält und der weiterhin 0,1–10 Gew.-% einer alkalisch reagierenden Alkalimetallverbindung enthält, wobei alle Prozentsätze auf das Gesamtgewicht des Katalysators bezogen sind, einsetzt und die Hydrierung bei 150–220°C und einem Druck von 0,5–10 bar, bevorzugt 0,5–4 bar, besonders bevorzugt 0,7–2 bar durchführt.

2. Verfahren nach Anspruch 1, dadurch gekennzeichnet, daß man zur Erhöhung des Anteils an Dicyclohexylamin eine niedrigere Temperatur innerhalb des Bereiches von 150–220°C wählt.

3. Verfahren nach Anspruch 1, dadurch gekennzeichnet, daß man eine Katalysatorbelastung von 0,05–2 kg, bevorzugt 0,1–0,5 kg Anilin pro Liter Katalysator und Stunde einstellt.

4. Verfahren nach Anspruch 1, dadurch gekennzeichnet, daß man ein Anilin der Formel

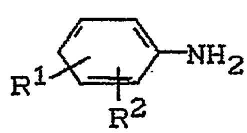

einsetzt, in der $R^1$ und $R^2$ unabhängig voneinander Wasserstoff, $C_1$–$C_4$-Alkyl oder $C_1$–$C_4$-Alkoxy bedeuten.

5. Verfahren nach Anspruch 1, dadurch gekennzeichnet, daß man einen Katalysator einsetzt, dessen Träger ein $Al_2O_3$ oder ein Aluminiumspinell ist.

6. Verfahren nach Anspruch 1, dadurch gekennzeichnet, daß der Katalysator vor seinem Einsatz mit Wasserstoff bei 120–400°C, bevorzugt 150–380°C, behandelt wird.

7. Verfahren nach Anspruch 1, dadurch gekennzeichnet, daß der Katalysator die Edelmetalle in einer Gesamtmenge von 0,1–4 Gew.-%, bevorzugt 0,1–3 Gew.-%, bezogen auf das Gesamtgewicht des Katalysators, enthält.

8. Verfahren nach Anspruch 1, dadurch gekennzeichnet, daß der Katalysator die Edelmetalle in einem Gewichtsverhältnis Ruthenium zu Palladium wie 2:8–8:2, bevorzugt 3:7–7:3, enthält.

9. Verfahren nach Anspruch 1, dadurch gekennzeichnet, daß der Katalysator 0,2–5 Gew.-%, einer basischen Alkalimetallverbindung, bezogen auf das Gesamtgewicht des Katalysators, enthält.

**Revendications**

1. Procédé pour la préparation d'un mélange constitué de cyclohexylamine éventuellement substituée et de dicyclohexylamine éventuellement substituée, par hydrogénation d'aniline éventuellement substituée, en présence d'un catalyseur de ruthénium, caractérisé en ce que l'on met en oeuvre un catalyseur contenant du ruthénium et du palladium, sur un support qui contient les métaux précieux dans une quantité totale de 0,05–5% en poids, le rapport pondéral du ruthénium au palladium étant de 1:9–9:1, et qui contient, en outre, 0,1–10% en poids d'un composé de métaux alcalins réagissant de manière alcaline, tous les pourcentages étant rapportés sur le poids total du catalyseur, et en ce qu'on procède à l'hydrogénation à 150–220°C et sous une pression de 0,5–10 bar, de préférence, de 0,5–4 bar, de manière particulièrement préférée, de 0,7–2 bar.

2. Procédé selon la revendication 1 caractérisé en ce que, en vue d'augmenter la fraction de dicyclohexylamine, on choisit une température inférieure au sein de l'intervalle allant de 150 à 220°C.

3. Procédé selon la revendication 1 caractérisé en ce qu'on met en oeuvre une sollicitation de catalyseur, de 0,05–2 kg, de préférence, de 0,1–0,5 kg d'aniline par litre de catalyseur et par heure.

4. Procédé selon la revendication 1 caractérisé en ce qu'on met en oeuvre une aniline de formule

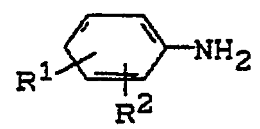

dans laquelle $R^1$ et $R^2$ représentent, indépendamment l'un de l'autre, un atome d'hydrogène, un groupe alkyle en $C_1$–$C_4$ ou un groupe alcoxy en $C_1$–$C_4$.

5. Procédé selon la revendication 1 caractérisé en ce qu'on met en oeuvre un catalyseur dont le support est un $Al_2O_3$ ou un spinelle d'aluminium.

6. Procédé selon la revendication 1 caractérisé en ce qu'on traite le catalyseur, avant sa mise en oeuvre, avec de l'hydrogène, à 120–400°C, de préférence, 150–380°C.

7. Procédé selon la revendication 1 caractérisé en ce que le catalyseur contient les métaux précieux en une quantité totale de 0,1–4% en poids, de préférence, 0,1–3% en poids, rapporté sur le poids total du catalyseur.

8. Procédé selon la revendication 1 caractérisé en ce que le catalyseur contient les métaux précieux dans un rapport pondéral ruthénium:palladium de 2:8–8:2, de préférence, 3:7–7:3.

9. Procédé selon la revendication 1 caractérisé en ce que le catalyseur contient 0,2–5 % en poids d'un composé basique de métaux alcalins, rapporté sur le poids total du catalyseur.

**Claims**

1. Process for the preparation of a mixture of substituted or unsubstituted cyclohexylamine and substituted or unsubstituted dicyclohexylamine by hydrogenation of substituted or unsubstituted aniline in the presence of a ruthenium catalyst, characterized in that the catalyst used is one containing ruthenium and palladium on a support containing the noble metals in a total amount of 0.05–5% by weight and a weight ratio of ruthenium to palladium such as 1:9–9:1 and furthermore containing 0.1–10% by weight of an alkaline alkali metal compound, all percentages being based on the total weight of the catalyst, and the hydrogenation is carried out at 150–220°C and at a pressure of 0.5–10 bar, preferably 0.5–4 bar, particularly preferably 0.7–2 bar.

2. Process according to Claim 1, characterized in that to increase the percentage of dicyclohexylamine a lower temperature within the range of 150–220°C is chosen.

3. Process according to Claim 1, characterized in that the space velocity of the catalyst is set to 0.05–2 kg, preferably 0.1–0.5 kg, of aniline per litre of catalyst and per hour.

4. Process according to Claim 1, characterized in that an aniline of the formula

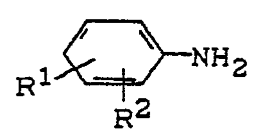

in which $R^1$ and $R^2$ independently of one another denote hydrogen, $C_1$–$C_4$-alkyl or $C_1$–$C_4$-alkoxy is used.

5. Process according to Claim 1, characterized in that a catalyst is used whose support is $Al_2O_3$ or an aluminium spinel.

6. Process according to Claim 1, characterized in that prior to its use the catalyst is treated with hydrogen at 120–400°C, preferably 150–380°C.

7. Process according to Claim 1, characterized in that the catalyst contains the noble metals in a total amount of 0.1–4% by weight, preferably 0.1–3% by weight, based on the total weight of the catalyst.

8. Process according to Claim 1, characterized in that the catalyst contains the noble metals in a weight ratio of ruthenium to palladium such as 2:8–8:2, preferably 3:7–7:3.

9. Process according to Claim 1, characterized in that the catalyst contains 0.2–5% by weight of a basic alkali metal compound, based on the total weight of the catalyst.